# EUROPEAN PATENT SPECIFICATION

(11) **EP 2 637 767 B1**
(45) Date of publication and mention of the grant of the patent: **26.04.2017**
(21) Application number: 11788270.4
(22) Date of filing: 08.11.2011
(51) Int. Cl.: B01D 53/14, B01D 53/86, C07C 51/44

(54) **PROCESSING GASEOUS STREAMS RESULTING FROM CARBONYLATION PROCESS**
VERARBEITUNG VON GASSTRÖMEN AUS CARBONYLIERUNGSVERFAHREN
FLUX DE GAZ DE TRANSFORMATION RÉSULTANT D'UN PROCÉDÉ DE CARBONYLATION

(30) Priority: 12.11.2010 US 413212 P
(43) Date of publication of application: 18.09.2013
(73) Proprietor: Eastman Chemical Company, Kingsport, TN 37660 (US)
(72) Inventor: LANE, Donald, Wayne, Kingsport TN 37663 (US); CONAWAY, Randall, Lee, Kingsport TN 37663 (US); PARTIN, Lee, Reynolds, Kingsport TN 37663 (US); BEWLEY, Jerry, Lee, Church Hill TN 37642 (US); JONES, William, Crawford, Seneca SC 29672 (US); BAYS, Joseph, Nathaniel, Kingsport TN 37664-4769 (US)
(74) Representative: Wibbelmann, Jobst
(86) International application number: PCT/US2011/059699
(87) International publication number: WO 2012/064689

(56) References cited:
- EP-A1- 0 075 730
- CN-A- 1 651 388
- US-A- 4 255 591
- US-A- 5 380 929
- US-A1- 2008 293 966

## Description

### BACKGROUND OF THE INVENTION

The preparation of acetic anhydride by contacting in the liquid phase a mixture containing methyl acetate and/or dimethyl ether and methyl iodide with carbon monoxide in the presence of a rhodium catalyst has been reported extensively. The process results in a number of gaseous streams that contain byproducts and unused portions of the raw materials. For example, in some flash separations, the product stream condenses and is thereby separated from gaseous streams that contain byproducts and unused raw materials. Gaseous components can also exist in carbonylation reactors, providing additional gaseous byproducts. Furthermore, equipment ancillary to carbonylation operations such as tanks and catalyst recovery processes also produce gaseous byproduct streams. These gaseous streams require further processing, and it is preferable to recover useful components of such gaseous streams. Because iodine-containing compounds such as methyl iodide are part of the feed to the carbonylation reactions, they are typically present in some of these gaseous streams. Recovering iodine-containing compounds from the gaseous streams is desirable for a number of reasons, including reducing the need to purchase further iodine-containing compounds and easing the burden presented by proper disposition of these byproducts. Thus, there is a continuing need in the art for improved efficiencies in recovering iodine-containing compounds from such gaseous streams.

Document EP 0 075 730 A1 discloses a method for recovering iodine-containing compounds from a gaseous, iodine compounds, methyl acetate and acetic acid containing byproducts stream recovered from a carbonylation process, which method comprises scrubbing the stream with methyl acetate containing liquid.

### BRIEF SUMMARY OF THE INVENTION

The invention provides a method according to claim 1 The method may further include a third scrubber step that includes contacting at least some of the acetic acid-scrubbed gaseous stream with a liquid containing water in a third scrubbing zone to produce a water-scrubbed gaseous stream and a third used scrubbant, the third used scrubbant containing water and acetic acid. The gaseous stream may also contain other components, for example hydrogen, carbon monoxide, acetic anhydride, nitrogen, ethylidene diacetate, methane, argon or a combination of two or more of the foregoing. Optionally, at least some of the third used scrubbant with reactants may be combined in a chemical process that can use a mixture of acetic acid and water.

In some embodiments, the method includes combining at least some of the first used scrubbant with carbonylation reactants in a carbonylation reaction zone, for example by combining the at least some first used scrubbant with one or more reactant feeds to the carbonylation reaction zone prior to the introduction of the reactant feeds to the carbonylation reaction zone. This may be done, for example, by doing the following: (a) processing at least some of the first used scrubbant in a first distillation zone to produce a first distillation overhead containing methyl acetate and at least one iodine-containing compound and a first distillation underflow containing methyl acetate: (b) combining at least some of the first distillation overhead with carbonylation reactants in the carbonylation reaction zone; and (c) recycling at least some of the first distillation underflow to the first scrubbing zone. In some embodiments, at least some first distillation overhead is combined with one or more reactant feeds to the carbonylation reaction zone prior to the introduction of the reactant feeds to the carbonylation reaction zone.

The process includes separating the second used scrubbant in a distillation zone to produce a second distillation overhead containing methyl acetate and a second distillation underflow containing acetic acid. At least some of the distillation underflow is recycled to the second scrubbing zone. In some embodiments, at least some of the distillation overhead may be combined with carbonylation reactants in a carbonylation reaction zone. This may be done by combining at least some of the distillation overhead with one or more reactant feeds to the carbonylation reaction zone prior to the introduction of the reactant feeds to the carbonylation reaction zone, feeding at least some of the distillation overhead to the first scrubbing zone, or both. In some embodiments the distillation overhead may be fed to the first distillation zone.

In some embodiments, the iodine-containing compound is selected from C1 - C12 alkyl iodides, elemental iodine, acetyl iodide, iodomethyl acetate, methyliodoacetate, iodoacetone, iodoacetic acid or any combination of two or more of the foregoing.

The invention allows use of a feed material (methyl acetate) as a scrubbant, but uses a second acetic acid scrubbing step to recover methyl acetate from the gas stream, thus overcoming the disadvantage of loss of a portion of the raw material. Acetic acid is likewise recovered and thus available for other uses. The invention further provides a variety of embodiments that allow reusing the used scrubbants and integrating them with carbonylation and other processes.

The invention provides a number of advantages that enhance process efficiency and integration. Methyl acetate is a very efficient scrubbant for many iodine-containing compounds, particularly methyl iodide. Because methyl iodide is used in methyl acetate carbonylation, the resulting scrubbant stream containing methyl acetate and methyl iodide can then be used in methyl acetate carbonylation as well as a number of other processes. Using acetic acid scrubbant to recovering methyl acetate from the resulting gas stream likewise recovers useful material and addresses the presence of methyl acetate, which is regulated as an air pollutant in some jurisdictions. Acetic acid is a very efficient scrubbant for methyl acetate and the resulting scrubbant stream also has a number of uses described below that allow integration in the overall acetyl process and enhanced efficiency. In embodiments where water is used as a third scrubbant, the invention further provides for recovery of acetic acid and generation of yet another stream that can be integrated in a variety of acetyl processes.

### BRIEF DESCRIPTION OF THE DRAWINGS

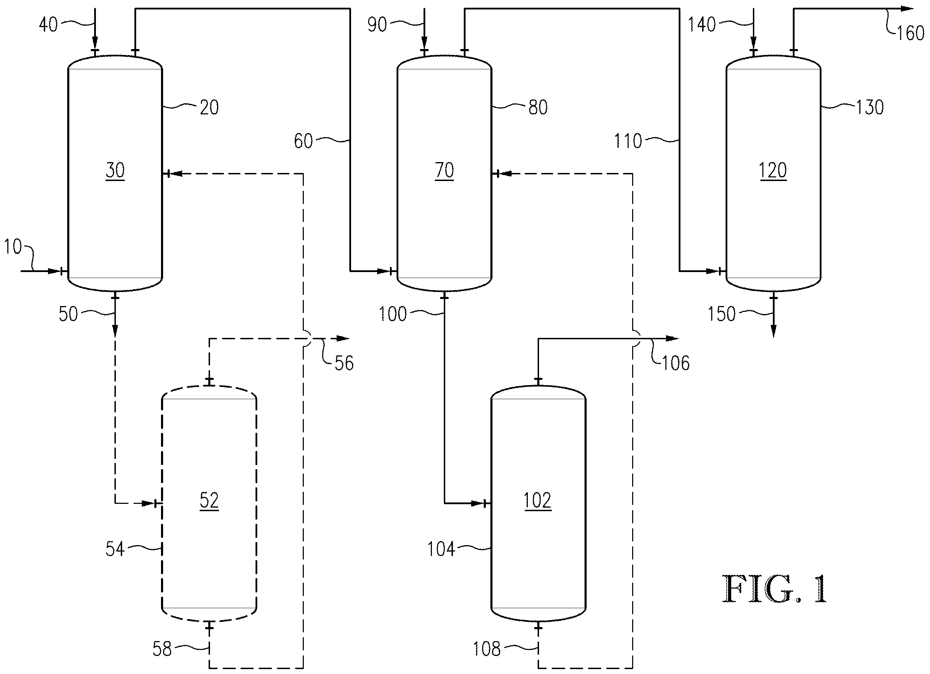

FIGURE 1 is a process diagram depicting certain embodiments of the present invention.

### DETAILED DESCRIPTION

The invention provides methods for recovering compounds from gaseous streams that result from carbonylation processes and ancillary processes. The carbonylation processes include carbonylation processes in which methyl acetate, dimethyl ether, or both reactants are fed to a reactor, optionally including methanol, along with other components such as catalysts, methyl iodide or other iodine-containing compounds, and solvents. The gaseous stream is processed in a series of at least two scrubbing zones. In the first scrubbing zone, the gas is scrubbed with a stream that contains methyl acetate to recover iodine-containing compounds in the scrubbant. The gas is then scrubbed in a second zone containing acetic acid to recover methyl acetate. Optionally, the gas is then placed into a third scrubbing zone where it is scrubbed with water. The used scrubbants may then be further processed or used in a variety of ways, including ways that allow integration with carbonylation and other chemical processes.

### Gaseous stream to be processed

The methods of the invention may be used to process gas streams resulting from carbonylation processes and ancillary equipment and processes. In some embodiments, some of the gas stream comes directly from one or more carbonylation reactors, either directly, via one or more flashing vessels downstream of the carbonylation reactor, or both. In some embodiments, the gaseous stream may include purge gasses or off-gasses from one or more related components or processes. Examples include gaseous streams from storage tanks associated with feed, intermediate or product storage, scrubbers associated with these storage tanks, condensers and vent condensers associated with product refining, vacuum systems, relief system equipment, and equipment associated with catalyst recovery. In some embodiments, the gas that enters the scrubbing train contains 0.5% to 50% carbon monoxide, 5 to 25% hydrogen, 5 to 50% nitrogen, 2 to 10% methyl acetate, 1 to 10% methane, 0.5 to 15% iodine-containing compounds, and traces of acetic acid, acetone, acetic anhydride, ethylidene diacetate, and inert gases present in air. Typical temperature and pressure of this combined vapor stream is near ambient and atmospheric conditions. Temperature may vary depending on temperature of cooling water in upstream condensations chillers, which is in turn affected by ambient conditions. Pressure may vary above and below ambient depending on the processes being controlled. Flow rates for the gaseous stream during normal operation may include a small fraction of the unreacted CO, impurities in gas feeds to the process, plus inert gas used for various purposes throughout the process. Flow rates may vary widely based on mode of operation, but in some embodiments, flow rates for the gaseous stream may vary from 30 to 200 kmol total vapor per hour.

In some embodiments the scrubbing system is used during times when the carbonylation reactor is not operational. Thus, offgasses from tanks, tank scrubbers, condensers and vent condensers, and catalyst recovery equipment are vented to the scrubbing system even when the process is not generating new carbonylation products. During these modes, the above composition numbers may vary widely beyond the ranges above. For example, CO levels may reach as high as 70% in some situations, and nitrogen levels may approach 100% during equipment purges during a shutdown.

### Iodine-containing compounds

As used throughout this application, "iodine-containing compounds" means any compounds that include at least one iodine atom. Examples include C1 - C12 alkyl iodides (e.g. methyl iodide, ethyl iodide, propionyl iodide, butyl iodide, etc.), elemental iodine, acetyl iodide, iodomethyl acetate, methyliodoacetate, iodoacetone, and iodoacetic acid. "Iodine-containing compounds" may also be a combination of any two or more of the foregoing. In some embodiments, the iodine-containing compound is one or more organic iodides. In some embodiments, the iodine-containing compound is one or more organic iodides having a sufficiently high volatility to be at least partially separated from methyl acetate by boiling. In some embodiments, the iodine-containing compound is one or more alkyl iodides. In some embodiments, the iodine-containing compounds include methyl iodide.

### Scrubbing steps

Each of the scrubbing steps takes place in a scrubbing zone. Any effective scrubbing zone may be used. In some embodiments the scrubbing zones may be separate vessels or two or more of the scrubbing zones may be disposed as parts of a single vessel. In each of the scrubbing steps, the gaseous stream is contacted with a liquid stream under conditions effective to scrub a desired portion of certain components from the gaseous streams. For each scrubbing step, the necessary flow of the scrubbant can be readily determined based on characteristics of the scrubbant and gas stream and the desired degree of scrubbing by standard engineering calculations or mathematical modeling using a simulation program such as those available from Aspen Technology, Inc., Burlington, Massachusetts. In some embodiments, a scrubbing step occurs at a temperature and pressure near ambient conditions, to make the scrubber both efficient and economical, without creating an economical penalty for more extreme conditions. Any effective scrubber geometry for providing effective contact between vapor and liquid may be used. If desired to increase scrubber efficiency, cooling can be provided to the scrubber by means of internal or external coolers, but this is not required.

Any effective type and configuration of scrubbing technologies can be used. Some examples of scrubbers include packed bed scrubbers, towers containing trays or packing, baffle tray scrubbers, spray towers and venturi scrubbers. Some examples of packing include Raschig rings, Pall rings and structured packing. Tray examples include valve trays, sieve trays, and bubble caps. The material of construction of the scrubber is not critical as any that can provide the needed structural stability and resistance to corrosion can be used. Some examples include stainless steel and nickel alloys. The scrubbers may or may not include liquid distribution components such as shower heads, spray nozzles, or liquid distribution trays.

The first scrubbing step uses as a scrubbant a liquid stream that contains methyl acetate. The flow rate of this liquid stream should be sufficient to recover the desired fraction of iodine-containing compounds. In some embodiments, the methyl acetate may include all or part of a methyl acetate feed stream to a process that uses both methyl acetate and iodine-containing compounds, such as a carbonylation process. The concentration of methyl acetate is not critical, and any effective concentration of methyl acetate may be used. In some embodiments, the scrubbant contains at least 50% methyl acetate. In some embodiments, the scrubbant contains at least 75% methyl acetate. In some embodiments, the scrubbant contains at least 90% methyl acetate. In some embodiments, the scrubbant contains at least 95% methyl acetate. The methyl acetate is effective to scrub at least a portion of iodine-containing compounds from the gaseous stream and into the liquid scrubbant. The scrubbant may contain other materials that do not undesirably detract from its ability to scrub iodine-containing compounds or lead to unwanted components in the used scrubbant or scrubbed gas streams. For example, the scrubbing liquid can be methyl acetate of commercially pure quality, containing 99% methyl acetate and less than 1% of either water or methanol. Alternatively, the scrubber can be methyl acetate which is not commercially pure, but of acceptable quality for use as feed in a carbonylation process, such as methyl acetate containing methanol as an impurity or a co-feed, in a concentration less than 5%. The result of this scrubbing step is a first used scrubbant that contains methyl acetate and iodine-containing compounds, and a methyl acetate-scrubbed gaseous stream. The gaseous stream contains gasses not removed by the scrubbing process, but also contains methyl acetate that has evaporated during the first scrubbing process (and may contain methyl acetate from the gaseous stream that entered the scrubber). The gaseous stream from the first scrubbing zone is transferred to the second scrubbing step.

The second scrubbing step uses a scrubbant that contains a carboxylic acid, a carboxylic acid anhydride or a mixture of the two. The concentration of acid and anhydride are not critical, and any effective concentration of acetic acid may be used. As used throughout this application, the term "acid/anhydride" in reference to a composition or a component of a composition shall be used to describe a composition or a component of a composition that is acetic acid, acetic anhydride, or a combination of the two, and the term "acetic acid/anhydride" in reference to a composition or a component of a composition shall be used to describe a composition or a component of a composition that is acetic acid, acetic anhydride, or a combination of the two. In some embodiments, the scrubbant contains at least 50% acetic acid/anhydride. In some embodiments, the scrubbant contains at least 75% acetic acid/anhydride. In some embodiments, the scrubbant contains at least 90% acetic acid/anhydride. In some embodiments, the scrubbant contains at least 95% acetic acid/anhydride. In some embodiments the acetic acid/anhydride is at least 70% acetic acid. In some embodiments the acetic acid/anhydride is at least 80% acetic acid. In some embodiments the acetic acid/anhydride is at least 90% acetic acid. In some embodiments the acetic acid/anhydride is at least 95% acetic acid. In some embodiments, the second scrubbant contains acetic acid. Other components may be present in amounts that do not undesirably interfere with the function of scrubbing system or create problems for the materials of construction. The acid-containing stream is effective to scrub at least a portion of methyl acetate from the gaseous stream. The flow rate of this liquid stream should be sufficient to recover the desired fraction of methyl acetate. The result is a second used scrubbant that contains methyl acetate and acetic acid, and an acetic acid-scrubbed gaseous stream. The gaseous stream contains gasses not removed by the scrubbing processes, but also contains acetic acid that has evaporated during the first scrubbing process (and may contain acetic acid from the gaseous stream that entered the scrubber). The gaseous stream from the second scrubbing zone is then transferred to the third scrubbing step.

In some embodiments, a third scrubbing step is employed, using a liquid containing water as a scrubbant. Water need not be pure or deionized, but should be free of contaminants that will compromise the scrubbing process or result in undesirably contaminated used scrubbant. The water is effective to scrub at least a portion of acetic acid/anhydride from the gaseous stream. The flow rate of this liquid stream should be sufficient to recover the desired fraction of acid/anhydride. The result is a third used scrubbant that contains water and acetic acid, and a water-scrubbed gaseous stream. The gaseous stream contains gasses not removed by the scrubbing process as well as water and any residual amounts of other scrubbants.

The water-scrubbed gaseous stream resulting from the third scrubbing step may be processed as desired. In some embodiments, the stream is disposed of, for example by burning or flaring. In some embodiments, the stream is processed further to recover additional gaseous components, such as carbon monoxide.

### Further processing of used scrubbants

Each of the scrubbing processes generates a used scrubbant, and the invention provides a variety of valuable ways to recycle or to otherwise use the scrubbants.

For example, the first used scrubbant from the methyl acetate scrubbing process contains methyl acetate and recovered iodine-containing compound. As such, it can be transferred to a process that uses methyl acetate and iodine-containing compounds, such as a carbonylation process. In some embodiments, the first used scrubbant may be subjected to a process to separate some of the methyl acetate from the used scrubbant stream. For example, the first used scrubbant can be processed in a first distillation zone to produce an overhead containing part of the methyl acetate and most or all of the iodine-containing compounds, and an underflow containing methyl acetate and lower levels of iodine-containing compounds. For example, in some embodiments the overhead stream can be refined to 40% or greater concentration of methyl iodide (*e.g.* 40-80%, 50-60%, *etc.*), with most of the balance of the composition being methyl acetate. while, the underflow stream may be reduced by appropriate distillation to an Mel content of 1 ppm or less, possibly even below detection limits. In such embodiments, the amount sent to the reactor feed can be reduced and the underflow can be recycled to the scrubber, thus reducing the overall methyl acetate demand for the scrubbing process.

The second used scrubbant from the acetic acid/anhydride scrubbing process contains acetic acid/anhydride and methyl acetate. As such, it can be transferred to any number of processes where a mixture of methyl acetate and acetic acid/anhydride can be used as feed. Some examples include carbonylation processes, processes that convert acetic acid to methyl acetate by reaction with methanol, and processes that hydrolyze methyl acetate to acetic acid and methanol. The second used scrubbant is subjected to a process to separate some of the acetic acid/anhydride from the used scrubbant stream. The second used scrubbant is processed in a distillation zone to produce an overhead containing part of the acetic acid/anhydride and concentrated methyl acetate, and an underflow containing higher concentrations of acetic acid/anhydride. Some of the underflow is recycled to the scrubber process to reduce the amount of demand of acetic acid/anhydride in the process. The overhead can be recycled to the methyl acetate scrubber or a location in the loop with the first scrubber and the first distillation column to reuse methyl acetate.

This invention can be further illustrated by the following examples, although it will be understood that these examples are included merely for purposes of illustration and are not intended to limit the scope of the invention unless otherwise specifically indicated.

### Some example embodiments demonstrated by the figure

Referring to Fig. 1, a stream 10 containing gasses from a carbonylation reactor and flash separation process as well as ancillary processes enters the bottom of a first scrubbing zone 30. In this embodiment, the first scrubbing zone 30 is contained in a single scrubber vessel 20, though this is not limiting. A liquid stream 40 containing 99% methyl acetate (and also containing small amounts of water, methanol and impurities) enters the top of first scrubbing zone 30 under conditions effective to allow scrubbing of at least some methyl iodide from the gaseous stream 10 into the liquid methyl acetate from the liquid stream 40. A liquid stream of used scrubbant 50 containing at least some of the methyl iodide and a methyl acetate-scrubbed gaseous stream 60 exit the scrubber. The entry points of streams 10 and 40 and exit points of 50 and 60 should not be viewed as limiting because configurations may vary depending on the type of scrubbing zone used.

The methyl acetate-scrubbed gaseous stream 60 then enters the bottom of a second scrubbing zone 70. In this embodiment the second scrubbing zone 70 is contained in a single scrubber vessel 80, though this is not limiting. A liquid stream 90 containing 95% acetic acid, 5% acetic anhydride (and small amounts of impurities) also enters the second scrubbing zone 70 under conditions effective to allow scrubbing of at least some methyl acetate from the gaseous methyl acetate-scrubbed gaseous stream 60 into the liquid acetic acid from the liquid stream 90. A liquid stream of used scrubbant 100 containing some of the methyl acetate and an acetic acid-scrubbed gaseous stream 110 exit the scrubber. The entry points of streams 60 and 90 and exit points 100 and 110 should not be viewed as limiting because configurations may vary depending on the type of scrubbing zone used.

The acetic acid-scrubbed gaseous stream 110 then enters the bottom of a third scrubbing zone 120. Optionally, the third scrubbing zone is a series of vertically arranged packed beds with water recirculating in each bed to increase liquid holdup to allow mass transfer to occur efficiently at a water feed rate that would be too low to be easily distributed on the surface of the packing. In this embodiment, the third scrubbing zone 120 is contained in a single scrubber vessel 130, though this is not limiting. An aqueous liquid stream 140 also enters the top of third scrubbing zone 120 under conditions effective to allow scrubbing of at least some acetic acid from the acetic acid-scrubbed gaseous stream 110 into the water from the aqueous liquid stream 140. A liquid stream of used scrubbant 150 containing some of the acetic acid and an acetic acid-scrubbed gaseous stream 160 exit the scrubber. The entry points of streams 110 and 140 and exit points 150 and 160 should not be viewed as limiting because configurations may vary depending on the type of scrubbing zone used. Although the three scrubbing zones 30, 70 and 120 are shown as appearing in separate vessels 20, 80 and 130, this arrangement is not limiting as two or more scrubbing zones can be configured within a single vessel.

The scrubbing processes are not limited to embodiments in which a specific amount of a component is removed, and can be operated to attain a variety of removal rates as desired.

This process thus yields three used scrubbant streams, 50, 100 and 150 that may each be processed further, used in other processes or both. Used scrubbant stream 50, containing methyl acetate and methyl iodide, may be fed to a carbonylation process for example by piping the material directly to a carbonylation zone (not pictured) or by piping the material into a storage vessel (not pictured) that contains one or more of the reactants or feeds to be used in the methyl carbonylation process.

Optionally, stream 50 can be separated into two separate streams in a distillation zone 52 to further concentrate the methyl iodide (and optionally other iodine-containing compounds) in a methyl acetate stream. In this figure, the distillation zone 52 is a column. While not shown in the figure, the column can optionally possess a reboiler at the bottom as well as reflux pot and condenser at the column head. The distillation is effective to concentrate the methyl acetate in the underflow stream 58 and the methyl iodide in the overhead stream 56, but the invention is not limited to embodiments in which the separation is complete, and embodiments exist in which methyl iodide is present in the underflow stream 58 and methyl acetate is present in the overhead stream 56. In some embodiments, the distillation of methyl iodide from methyl acetate is very temperature sensitive such that it may be useful to maintain a relatively flat temperature. The relative concentrations in the streams will depend in part on the design and operation parameters of the distillation process, such as number of trays, reflux ratio, and the desired concentrations of streams 56 and 58. In this embodiment the distillation zone 52 is contained in a vessel 54, such as a distillation column, though this is not limiting. In this optional embodiment, stream 56 may then be fed to or combined with feed to a carbonylation process (not pictured). Optionally, stream 58 is recycled (as pictured) to the first scrubbing zone 30. In some embodiments, stream 58 is combined with the methyl acetate liquid feed stream 40 to first scrubbing zone 30. Optionally, stream 58 may be used in any process (not pictured) in which a methyl acetate stream having a higher purity of methyl acetate than stream 50 is desired. In some embodiments, stream 58 is a stream containing 85-95% methyl acetate, 5-15% acetic acid, and less than 1 ppm methyl iodide.

In some embodiments where stream 50 is processed in column 52, a purge (not shown) of stream 50 is taken off and combined with the feed to a carbonylation reaction to remove higher boilers, such as acetic acid, from the loop made up of scrubbing zone 30 and distillation zone 52 and their associated piping. In some embodiments, this purge is 1-10% of the total volume passing through the loop, but this is not limiting.

Stream 100, containing methyl acetate in acetic acid may, for example, be separated in a distillation zone 102 to result in an underflow stream 108 containing acetic acid and an overhead stream 106 containing methyl acetate. In this figure, the distillation zone 102 is a column. While not shown in the figure, the column can optionally possess a reboiler at the bottom as well as reflux pot and condenser at the column head. The distillation is effective to concentrate the acetic acid in the underflow stream 108 and the methyl acetate in the overhead stream 106, but the invention is not limited to embodiments in which the separation is complete, and embodiments exist in which methyl acetate is present in the underflow stream 108 and acetic acid is present in the overhead stream 106. The relative concentrations in the streams will depend in part on the design and operation parameters of the distillation process, such as number of trays, reflux ratio and desired concentrations of streams 108 and 106. In this embodiment, the distillation zone 102 is contained in a vessel 104, such as a distillation column, though this is not limiting. The methyl acetate and acetic acid streams may be used in any desirable manner. In some embodiments, the underflow stream 108 is fed to scrubbing zone 70 as shown in Fig 1. In some embodiments (not pictured), underflow stream 108 may be combined with stream 90 to feed scrubbing zone 70. In some embodiments, the overhead stream 106 is fed to a process that uses acetic acid. In some embodiments, the underflow stream containing acetic acid 108 is fed to a process wherein acetic acid is reacted with methanol to produce methyl acetate. In some embodiments, the overhead stream 106 is fed to a carbonylation process, for example by piping the material directly to a carbonylation zone (not pictured) or by piping the material into a storage vessel (not pictured) that contains one or more of the reactants or feeds to be used in the methyl carbonylation process. In some embodiments, the overhead stream 106 is fed to scrubbing zone 30. In some embodiments, the overhead stream 106 is combined with methyl acetate stream 40 fed to scrubbing zone 30. In some embodiments, the overhead stream 106 is combined with stream 50 or fed to distillation zone 52.

Stream 150, containing water and acetic acid, may be used in any process (not pictured) where a dilute stream of acetic acid in water is useful. Some examples include processes for production of acetic acid through hydrolysis of acetic anhydride and processes for production of esters from alcohols and carboxylic acid (*e*.*g*. processes wherein acetic acid is reacted with methanol to produce methyl acetate, or butanol to product butyl acetate).

In some embodiments, one or more of the feeds to the scrubbers are chilled to improve scrubbing efficiency. For example, the methyl acetate feed to the first scrubber may be cooled to a temperature of between 5 and 30°C, in some embodiments between 15 and 25°C. As another example, the acetic acid feed to the second scrubber may be cooled to a temperature above the freezing point up to 30°C, in some embodiments between 20 and 25°C. Recycle streams may also be cooled in some embodiments. Any recycle stream may be cooled if the cooling imparts a benefit to the scrubbing efficiency of the system. For example, the underflow stream 58 from column 52 may be cooled to a temperature of between 5 and 30°C, in some embodiments between 15 and 25°C before entering scrubber 30. As another example, the underflow stream 108 from column 102 may be cooled to a temperature above the freezing point up to 50°C, before entering the scrubber 70. In some embodiments, the acetic acid feed to the second scrubber and the underflow stream 108 are first mixed and then cooled to a temperature above the freezing point up to 50°C before entering the scrubber 70.

### EXAMPLES

### EXAMPLE 1

In the following example, selected flow rates are provided on both volumetric (volumes per minute) and mass (parts per minute) bases. As a basis for correlating these measures, it may be assumed that liquid water at 25°C would have a density of about 0.9947 mass parts per volume.

A gaseous stream from a methyl acetate carbonylation reactor, flash train, and related equipment contained 28% iodine-containing compounds, 34% carbon monoxide, 24% methyl acetate, 8% nitrogen, 4% acetone, less than 1% or undetectable levels for each of hydrogen, acetic acid, acetic anhydride, ethylidene diacetate and methane. The stream was inserted into the bottom of a packed bed scrubber having two packed beds. The bottom bed packing was 1.5 inch diameter 316L stainless steel Pall Rings and the top bed was packed with two packings. The lower fourth of the top packed bed was filled with 1.5 inch diameter Hastelloy G Pall rings and the top three quarters was filled with 1.5 inch diameter 316L stainless steel Pall Rings. The column was operated under vacuum at 735 torr and at ambient temperature. The gas was scrubbed with 98% methyl acetate with a flow rate of 100 volumes per minute. The used scrubbant liquid containing approximately 95% methyl acetate and 4% methyl iodide was transported to a feed tank for a carbonylation reactor for reuse in the carbonylation reactor.

The methyl acetate-scrubbed gaseous stream flowing at a rate of about 27.2 parts per minute from the first packed scrubber was then inserted into the bottom of a second packed bed scrubber where it was scrubbed with a liquid stream containing 98% acetic acid and 2% acetic anhydride. This scrubber contained three vertically arranged beds. The bottom packed bed was packed with the IMTP® 50 316L stainless steel random packing and the middle and top beds were packed with the IMTP® 25 316L stainless steel random packing. For each packed bed, a liquid distributor was installed which was designed for the intended liquid flowrate at that location. The scrubbant stream was fed at a normal rate of 50 volumes per minute into the top of the column at ambient temperature. A large recirculation system was set up to return 250 volumes per minute of liquid from the bottom of the scrubber to a feed point between the bottom and middle packed beds to cool the scrubber, which improved its efficiency. The stream temperature was controlled at 25°C.

The used scrubbant from the second scrubber containing 11% methyl acetate, 87% acetic acid and 2% acetic anhydride was inserted at a normal rate of 50 volumes per minute, in the middle of a packed distillation column having an underflow rate of 26 volumes per minute, a distillate flow rate of 24 volumes per minute and a reflux rate of 12 volumes per minute. The column included three packed beds with 1.5" 316L stainless steel pall rings and operated a base temperature of 100 to 125 degrees Celsius and a top temperature of 90 to 110 degrees Celsius. The overhead from the distillation column containing 48% methyl acetate and 50% acetic acid was piped to a feed tank for a carbonylation reactor for reuse in the carbonylation process. The underflow from the first distillation column containing approximately 1% methyl acetate and 97% acetic acid was recycled as part of the acetic acid feed to the middle of to the second packed bed scrubber.

An acetic acid-scrubbed gaseous stream from the second packed scrubber was then inserted into the bottom of a third packed bed scrubber at a rate of about 16 parts per minute where it was scrubbed with a stream of process water fed at about 3 to 5 volumes per minute to the top of the scrubber. This was a single bed scrubber with 1.5 inch diameter 316L stainless steel pall ring packing. The water-scrubbed gaseous stream was then burned. The used scrubbant from the third scrubber containing 70% water and 29% acetic acid and methyl acetate was combined with the feed for a reactive distillation column for producing methyl acetate from methanol and acetic acid or for hydrolysis of acetic anhydride to acetic acid.

### EXAMPLE 2

The configuration of Example 1 is employed, except that the used scrubbant from the first scrubber, containing methyl acetate and methyl iodide, is separated in a first distillation column into an overhead stream containing the majority (e.g. more than 99%) of the methyl iodide in methyl acetate and an underflow stream containing methyl acetate. The column is operated with an approximate reflux ratio of 75, has 30 theoretical stages (excluding condenser and reboiler) and is packed with IMTP 40 millimeter random packing. The packing is arrayed in beds that have hastelloy shells and packing toward the top of the column and stainless steel toward the bottom. This allows concentration of methyl iodide in the overhead stream, which is piped to a feed tank for a carbonylation reactor for reuse in the carbonylation process. As a result, the underflow from the first distillation column containing about 99% methyl acetate is fed to the first packed bed scrubber, thus reducing the amount of makeup methyl acetate that is needed in the scrubber. This produces an underflow in which methyl iodide value was below detection limit. The overhead has methyl iodide levels between 50 and 60% with the balance being MeOAc.

Optionally, the overhead stream containing methyl acetate from the distillation column (concentration of methyl acetate from acetic acid scrubbant) is combined with the first distillation process. For example, the stream may be combined with the used scrubbant stream from the first scrubber before entry into the first distillation column, piped directly to the first distillation column, or combined with the purified methyl acetate stream from the underflow of the first distillation column.

In the drawing and specification, there have been disclosed typical preferred embodiments of the invention and, although specific terms are employed, they are used in a generic and descriptive sense only and not for purposes of limitation, the scope of the invention being set forth in the following claims.

## Claims

1. A method for recovering iodine-containing compounds from a gaseous byproducts stream comprising at least one iodine-containing compound, the method comprising:
receiving gaseous byproducts comprising at least one iodine containing compound, methyl acetate, and acetic acid from a carbonylation process,
contacting a gaseous stream comprising said gaseous byproducts comprising said at least one iodine containing compound, methyl acetate, and acetic acid with a liquid comprising methyl acetate in a first scrubbing zone to produce a methyl acetate-scrubbed gaseous stream and a first used scrubbant, the first used scrubbant comprising methyl acetate, acetic acid, and the iodine-containing compound; and
contacting at least some of the methyl acetate-scrubbed gaseous stream with a liquid comprising acetic acid in a second scrubbing zone to produce an acetic acid-scrubbed gaseous stream and a second used scrubbant, the second used scrubbant comprising methyl acetate and acetic acid; and wherein
the method further comprises separating the second used scrubbant in a distillation zone to produce a distillation overhead comprising methyl acetate and a distillation underflow comprising acetic acid, wherein at least some of the distitiation underflow is recycled to the second scrubbing zone and wherein the distillation overhead is further processed in the carbonylation process and/or is used in other processes.

2. The method of claim 1, wherein said gaseous byproducts stream comprises gaseous components obtained from a carbonylation reactor, flashing vessels downstream of a carbonylation reactor, purge gasses or off-gasses from one or more components or processes related to the carbonylation process, gaseous streams from storage tanks associated with feed, intermediate or product storage for the carbonylation process, condensers and vent condensers associated with carbonylation product refining, vacuum systems, relief system equipment, equipment associated with catalyst recovery, or combinations of two or more of the foregoing.

3. The method of claim 1 or 2, wherein the method further comprises contacting at least some of the acetic acid-scrubbed gaseous stream with a liquid comprising water in a third scrubbing zone to produce a water-scrubbed gaseous stream and a third used scrubbant, the third used scrubbant comprising water and acetic acid.

4. The method of claim 3, further comprising combining at least some of the third used scrubbant with reactants in a chemical process that can use a mixture of acetic acid and water.

5. The method of claim 4 wherein the chemical process that can use a mixture of acetic acid and water
(i) is selected from processes for production of esters from alcohols and carboxylic acid and processes for production of acetic acid through hydrolysis of acetic anhydride, or
(ii) is a process for the production of methyl acetate from methanol and acetic acid.

6. The method of any of claims 1-5 wherein the gaseous byproducts stream further comprises one or more components selected from hydrogen, carbon monoxide, acetic anhydride, nitrogen, ethylidene diacetate, methane, argon or a combination of two or more of the foregoing.

7. The method of any of claims 1-6, further comprising combining at least some of the first used scrubbant with carbonylation reactants in a carbonylation reaction zone, preferably wherein combining at least some of the first used scrubbant with carbonylation reactants in a carbonylation reaction zone comprises combining the at least some first used scrubbant with one or more reactant feeds to the carbonylation reaction zone prior to the introduction of the reactant feeds to the carbonylation reaction zone.

8. The method of claim 7, wherein combining at least some of the first used scrubbant with carbonylation reactants in a carbonylation reaction zone comprises:
processing at least some of the first used scrubbant in a first distillation zone to produce a first distillation overhead comprising methyl acetate and at least one iodine-containing compound and a first distillation underflow comprising methyl acetate;
combining at least some of the first distillation overhead with carbonylation reactants in the carbonylation reaction zone; and
recycling at least some of the first distillation underflow to the first scrubbing zone, preferably wherein combining at least some of the first distillation overhead with carbonylation reactants in the carbonylation reaction zone comprises combining the at least some first distillation overhead with one or more reactant feeds to the carbonylation reaction zone prior to the introduction of the reactant feeds to the carbonylation reaction zone.

9. The method of claim 1, further comprising combining at least some of the distillation overhead from the distillation of the second used scrubbant with carbonylation reactants in a carbonylation reaction zone, preferably wherein combining at least some of the distillation overhead with carbonylation reactants in a carbonylation reaction zone comprises combining the at least some distillation overhead with one or more reactant feeds to the carbonylation reaction zone prior to the introduction of the reactant feeds to the carbonylation reaction zone.

10. The method of claim 8 or 1, further comprising feeding at least some of the distillation overhead from the distillation of the second used scrubbant to the first scrubbing zone or to the first distillation zone.

11. The method of any of claims 1-10, wherein the at least one iodine-containing compound is selected from C1 - C12 alkyl iodides, elemental iodine, acetyl iodide, iodomethyl acetate, methyliodoacetate, iodoacetone, iodoacetic acid or any of combination of two or more of the foregoing.

12. The method of any of claims 1-11, wherein the at least one iodine-containing compound comprises methyl iodide.

13. A carbonylation process for producing acetic anhydride, comprising a method for recovering iodine-containing compounds from a gaseous byproducts stream as defined in any of claims 1-12.

## Patentansprüche

1. Verfahren zum Gewinnen von Iod-enthaltenden Verbindungen aus einem gasförmigen Nebenprodukte-Strom, umfassend mindestens eine Iod-enthaltende Verbindung, wobei das Verfahren umfasst:
Erhalten von gasförmigen Nebenprodukten, umfassend mindestens eine Iod-enthaltende Verbindung, Methylacetat und Essigsäure aus einem Carbonylierungsprozess,
In-Kontakt-Bringen eines gasförmigen Stroms, umfassend diese gasförmigen Nebenprodukte, umfassend diese mindestens eine Iod-enthaltende Verbindung, Methylacetat und Essigsäure, mit einer Flüssigkeit, umfassend Methylacetat in einer ersten Waschzone, um einen Methylacetat-gewaschenen gasförmigen Strom und ein erstes gebrauchtes Waschmittel zu erzeugen, wobei das erste gebrauchte Waschmittel Methylacetat, Essigsäure und die Iod-enthaltende Verbindung umfasst; und
In-Kontakt-Bringen von zumindest etwas des Methylacetat-gewaschenen Stroms mit einer Flüssigkeit, umfassend Essigsäure in einer zweiten Waschzone, um einen Essigsäure-gewaschenen gasförmigen Strom und ein zweites gebrauchtes Waschmittel zu erzeugen, wobei das zweite gebrauchte Waschmittel Methylacetat und Essigsäure umfasst; und wobei
das Verfahren weiterhin das Abtrennen des zweiten gebrauchten Waschmittels in einer Destillationszone umfasst, um eine Destillationskopffraktion, umfassend Methylacetat und einen Destillationsunterlauf, umfassend Essigsäure, zu erzeugen, wobei zumindest etwas des Destillationsunterlaufs in die zweite Waschzone recycelt wird und wobei die Destillationskopffraktion in dem Carbonylierungsprozess weiterverarbeitet und/oder in anderen Prozessen verwendet wird.

2. Das Verfahren nach Anspruch 1, wobei dieser gasförmige Nebenprodukte-Strom gasförmige Komponenten umfasst, bezogen aus einem Carbonylierungsreaktor, Flash-Behältern stromabwärts eines Carbonylierungsreaktors, Spülgassen oder Abgasen von einer oder mehreren Komponenten oder Prozessen, die in Beziehung mit dem Carbonylierungsprozess stehen, gasförmigen Strömen aus Speichertanks, die mit der Zufuhr assoziiert sind, Zwischenprodukt- oder Produktlagerung für den Carbonylierungsprozess, Kondensatoren und Entlüftungskondensatoren, die mit der Raffination des Carbonylierungsprodukts assoziiert sind, Vakuumsystemen, Entlastungssystemausrüstung, Ausrüstung, die mit Katalysatorgewinnung assoziiert ist, oder Kombinationen aus zwei oder mehreren der Vorangehenden.

3. Das Verfahren nach Anspruch 1 oder 2, wobei das Verfahren weiterhin das In-Kontakt-Bringen von zumindest etwas des Essigsäure-gewaschenen gasförmigen Stroms mit einer Flüssigkeit umfassend Wasser, in einer dritten Waschzone, umfasst, um einen Wasser-gewaschenen gasförmigen Strom und ein drittes gebrauchtes Waschmittel zu erzeugen, wobei das dritte gebrauchte Waschmittel Wasser und Essigsäure umfasst.

4. Das Verfahren nach Anspruch 3, weiterhin umfassend das Kombinieren von zumindest etwas des dritten gebrauchten Waschmittels mit Reaktanden in einem chemischen Prozess, der eine Mischung aus Essigsäure und Wasser verwenden kann.

5. Das Verfahren nach Anspruch 4, wobei der chemische Prozess, der eine Mischung aus Essigsäure und Wasser verwenden kann,
(i) ausgewählt ist aus Prozessen zur Herstellung von Estern von Alkoholen und Carbonsäure und Prozessen zur Herstellung von Essigsäure durch Hydrolyse von Essigsäureanhydrid, oder
(ii) ein Prozess für die Herstellung von Methylacetat aus Methanol und Essigsäure ist.

6. Das Verfahren nach einem beliebigen der Ansprüche 1-5, wobei der gasförmige Nebenprodukte-Strom weiterhin eine oder mehrere Komponenten, ausgewählt aus Wasserstoff, Kohlenstoffmonoxid, Essigsäureanhydrid, Stickstoff, Ethylidendiacetat, Methan, Argon oder einer Kombination von zwei oder mehreren der Vorangegangenen, umfasst.

7. Das Verfahren nach einem beliebigen der Ansprüche 1-6, weiterhin umfassend das Kombinieren von zumindest etwas des ersten gebrauchten Waschmittels mit Carbonylierungsreaktanden in einer Carbonylierungs-Reaktionszone, wobei bevorzugt das Kombinieren von zumindest etwas des ersten gebrauchten Waschmittels mit den Carbonylierungsreaktanden in einer Carbonylierungs-Reaktionszone das Kombinieren von zumindest etwas des ersten gebrauchten Waschmittels mit einer oder mehreren Reaktanden-Einspeisung(en) in der Carbonylierungs-Reaktionszone vor der Einführung der Reaktanden-Einspeisung in der Carbonylierungs-Reaktionszone, umfasst.

8. Das Verfahren nach Anspruch 7, wobei das Kombinieren von zumindest etwas des ersten gebrauchten Waschmittels mit den Carbonylierungsreaktanden in einer Carbonylierungs-Reaktionszone umfasst:
Verarbeiten von zumindest etwas des ersten gebrauchten Waschmittels in einer ersten Destillationszone, um eine erste Destillationskopffraktion, umfassend Methylacetat und mindestens eine Iod-enthaltende Verbindung und einen ersten Destillationsunterfluss, umfassend Methylacetat, zu erzeugen;
Kombinieren von zumindest etwas der ersten Destillationskopffraktion mit Carbonylierungsreaktanden in der Carbonylierungs-Reaktionszone; und
Recyceln von zumindest etwas des ersten Destillationsunterflusses zu der ersten Waschzone, wobei bevorzugt das Kombinieren von zumindest etwas der ersten Destillationskopffraktion mit Carbonylierungsreaktanden in der Carbonylierungs-Reaktionszone das Kombinieren des zumindest etwas der ersten Destillationskopffraktion mit einer oder mehreren Reaktanden-Einspeisung(en) zu der Carbonylierungs-Reaktionszone vor dem Einführen der Reaktanden-Einspeisungen in die Carbonylierungs-Reaktionszone, umfasst.

9. Das Verfahren nach Anspruch 1, weiterhin umfassend das Kombinieren von zumindest etwas der Destillationskopffraktion von der Destillation des zweiten gebrauchten Waschmittels mit Carbonylierungsreaktanden in einer Carbonylierungs-Reaktionszone, wobei bevorzugt das Kombinieren von zumindest etwas der Destillationskopffraktion mit Carbonylierungsreaktanden in einer Carbonylierungs-Reaktionszone das Kombinieren von zumindest etwas der Destillationskopffraktion mit einer oder mehreren Reaktanden-Einspeisung(en) zu der Carbonylierungs-Reaktionszone vor dem Einführen der Reaktanden-Einspeisungen in die Carbonylierungs-Reaktionszone, umfasst.

10. Das Verfahren nach Anspruch 8 oder 1, weiterhin umfassend das Zuführen von zumindest etwas der Destillationskopffraktion von der Destillation des zweiten gebrauchten Waschmittels zu der ersten Waschzone oder zu der ersten Destillationszone.

11. Das Verfahren nach einem beliebigen der Ansprüche 1-10, wobei die mindestens eine Iod-enthaltende Verbindung ausgewählt ist aus C1-C12-Alkyliodiden, elementarem Iod, Acetyliodid, Iodmethylacetat, Methyliodacetat, Iodaceton, Iod-Essigsäure oder eine beliebige Kombination von zwei oder mehreren der Vorangehenden.

12. Das Verfahren nach eine beliebigen der Ansprüche 1-11, wobei die mindestens eine Iod-enthaltende Verbindung Methyliodid umfasst.

13. Carbonylierungsprozess zur Herstellung von Essigsäureanhydrid, umfassend ein Verfahren zum Gewinnen von Iod-enthaltenden Verbindungen aus einem gasförmigen Nebenprodukte-Strom, wie in einem beliebigen der Ansprüche 1-12 definiert.

## Revendications

1. Procédé de récupération de composés contenant de l'iode à partir d'un courant de sous-produits gazeux comprenant au moins un composé contenant de l'iode, le procédé comprenant :
la réception de sous-produits gazeux comprenant au moins un composé contenant de l'iode, d'acétate de méthyle et d'acide acétique provenant d'un procédé de carbonylation,
la mise en contact d'un courant gazeux comprenant lesdits sous-produits gazeux comprenant ledit ou lesdits composés contenant de l'iode, l'acétate de méthyle et l'acide acétique avec un liquide comprenant de l'acétate de méthyle dans une première zone d'épuration pour produire un courant gazeux épuré à l'acétate de méthyle et un premier épurateur usagé, le premier épurateur usagé comprenant de l'acétate de méthyle, de l'acide acétique et le composé contenant de l'iode; et
la mise en contact d'au moins une partie du courant gazeux épuré à l'acétate de méthyle avec un liquide comprenant de l'acide acétique dans une deuxième zone d'épuration pour produire un courant gazeux épuré à l'acide acétique et un deuxième épurateur usagé, le deuxième épurateur usagé comprenant de l'acétate de méthyle et de l'acide acétique ; et dans lequel
le procédé comprend en outre la séparation du deuxième épurateur usagé dans une zone de distillation pour produire un distillat de tête comprenant de l'acétate de méthyle et un flux inférieur de distillation comprenant de l'acide acétique, dans lequel au moins un partie du flux inférieur de distillation est recyclée vers la deuxième zone d'épuration et dans lequel le distillat de tête est encore traité dans le procédé de carbonylation et/ou est utilisé dans d'autres procédés.

2. Procédé selon la revendication 1, dans lequel ledit courant de sous-produits gazeux comprend des constituants gazeux obtenus à partir d'un réacteur de carbonylation, de récipients de vaporisation instantanée en aval d'un réacteur de carbonylation, de gaz de purge ou de gaz d'échappement provenant d'un ou de plusieurs composants ou procédés liés au procédé de carbonylation, de courants gazeux provenant de cuves de stockage associées au stockage de l'alimentation, d'intermédiaire ou de produit pour le procédé de carbonylation, de condenseurs et de condenseurs d'évacuation associés à des systèmes sous vide, de raffinage de produit de carbonylation, à un équipement de système d'allègement, à un équipement associé à la récupération d'unu catalyseur ou des combinaisons de deux ou plus des éléments précités.

3. Procédé selon la revendication 1 ou 2, dans lequel le procédé comprend en outre la mise en contact d'au moins une partie du courant gazeux épuré à l'acide acétique avec un liquide comprenant de l'eau dans une troisième zone d'épuration pour produire un courant gazeux épuré à l'eau et un troisième épurateur usagé, le troisième épurateur usagé comprenant de l'eau et de l'acide acétique.

4. Procédé selon la revendication 3, comprenant en outre la combinaison d'au moins une partie du troisième épurateur usagé avec des réactifs dans un procédé chimique qui peut utiliser un mélange d'acide acétique et d'eau.

5. Procédé selon la revendication 4, dans lequel le procédé chimique qui peut utiliser un mélange d'acide acétique et d'eau
(i) est choisi parmi des procédés de production d'esters à partir d'alcools et d'acide carboxylique et des procédés de production d'acide acétique par hydrolyse d'anhydride acétique, ou
(ii) est un procédé de production d'acétate de méthyle à partir de méthanol et d'acide acétique.

6. Procédé selon l'une quelconque des revendications 1 à 5, dans lequel le courant de sous-produits gazeux comprend en outre un ou plusieurs constituants choisis parmi l'hydrogène, le monoxyde de carbone, l'anhydride acétique, l'azote, le diacétate d'éthylidène, le méthane, l'argon ou une combinaison de deux ou plus des éléments précités.

7. Procédé selon l'une quelconque des revendications 1 à 6, comprenant en outre la combinaison d'au moins une partie du premier épurateur usagé avec des réactifs de carbonylation dans une zone de réaction de carbonylation, dans lequel de préférence la combinaison d'au moins une partie du premier épurateur usagé avec les réactifs de carbonylation dans une zone de réaction de carbonylation comprend la combinaison de la au moins une partie du premier épurateur usagé avec un ou plusieurs réactifs alimentés à la zone de réaction de carbonylation avant l'introduction du réactif alimenté à la zone de réaction de carbonylation.

8. Procédé selon la revendication 7, dans lequel la combinaison d'au moins une partie du premier épurateur usagé avec des réactifs de carbonylation dans une zone de réaction de carbonylation comprend :
le traitement d'au moins une partie du premier épurateur usagé dans une première zone de distillation pour produire un premier distillat de tête comprenant de l'acétate de méthyle et au moins un composé contenant de l'iode et un premier flux inférieur de distillation comprenant de l'acétate de méthyle ;
la combinaison d'au moins une partie du premier distillat de tête avec des réactifs de carbonylation dans la zone de réaction de carbonylation ; et
le recyclage d'au moins une partie du premier flux inférieur de distillation vers la première zone d'épuration, dans lequel de préférence la combinaison d'au moins une partie du premier distillat de tête avec des réactifs de carbonylation dans la zone de réaction de carbonylation comprend la combinaison de la au moins une partie du premier distillat de tête avec un ou plusieurs réactifs alimentés à la zone de réaction de carbonylation avant l'introduction du réactif alimenté à la zone de réaction de carbonylation.

9. Procédé selon la revendication 1, comprenant en outre la combinaison d'au moins une partie du distillat de tête provenant de la distillation du deuxième épurateur usagé avec des réactifs de carbonylation dans une zone de réaction de carbonylation, dans lequel de préférence la combinaison d'au moins une partie du distillat de tête avec des réactifs de carbonylation dans une zone de réaction de carbonylation comprend la combinaison de la au moins une partie du distillat de tête avec un ou plusieurs réactifs alimentés à la zone de réaction de carbonylation avant l'introduction du réactif alimenté à la zone de réaction de carbonylation.

10. Procédé selon la revendication 8 ou 1, comprenant en outre l'alimentation d'au moins une partie du distillat de tête provenant de la distillation du deuxième épurateur usagé à la première zone d'épuration ou à la première zone de distillation.

11. Procédé selon l'une quelconque des revendications 1 à 10, dans lequel le ou les composés contenant de l'iodé sont choisis parmi les iodures d'alkyle en C1-C12, l'iode élémentaire, l'iodure d'acétyle, l'acétate d'iodométhyle, l'iodoacétate de méthyle, l'iodoacétone, l'acide iodoacétique ou l'une quelconque des combinaisons de deux ou plus des éléments précités.

12. Procédé selon l'une quelconque des revendications 1 à 11, dans lequel le ou les composés contenant de l'iode comprennent l'iodure de méthyle.

13. Procédé de carbonylation pour produire de l'anhydride acétique, comprenant un procédé de récupération de composés contenant de l'iode à partir d'un courant de sous-produits gazeux tel que défini selon l'une quelconque des revendications 1 à 12.
